(19) Europäisches Patentamt
European Patent Office
Office européen des brevets

(11) Publication number: 0 201 784 B1

(12) # EUROPEAN PATENT SPECIFICATION

(45) Date of publication of patent specification: 05.12.90

(51) Int. Cl.$^5$: A 61 K 35/78

(21) Application number: 86105751.1

(22) Date of filing: 25.04.86

(54) **Pharmaceutical composition decreasing side effects of anticancer chemotherapy and increasing the immune function.**

(30) Priority: 09.05.85 US 732146

(43) Date of publication of application:
20.11.86 Bulletin 86/47

(45) Publication of the grant of the patent:
05.12.90 Bulletin 90/49

(84) Designated Contracting States:
AT BE CH DE FR GB IT LI LU NL SE

(56) References cited:
GB-A-2 035 082

Deutsche Apotheker-Zeitung, 116. Jg. 1976, Stuttgart, DE, Nr. 1/2, pages 1-7

(73) Proprietor: Liu, Yaguang
67-08 168th Street
Fresh Meadows, NY 11365 (US)

(72) Inventor: Liu, Yaguang
67-08 168th Street
Fresh Meadows, NY 11365 (US)

(74) Representative: Diehl, Hermann O. Th., Dr. et al
Diehl & Partner Flüggenstrasse 13
D-8000 München 19 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to a new pharmaceutical composition for decreasing side effects of anticancer chemotherapy and for increasing the immune function in human beings.

A lot of anticancer medicines including chemical and antibiotics have effect to kill cancer cells. But they also kill some normal human cells. For example, Cyclophosphamide is a chemotherapeutic drug which is highly effective against a wide range of human cancer. Cyclophosphamide has established a role in the treatment of some major cancer types including Lymphomas, Acute Lymphatic Leukemias, Chronic Lymphatic Leukemias, Breast, Pulmonal, Ovarial cancer and Tumor of marrow mulciple, Osseous and Sarcoma. Unfortunately, Cyclophoshamide has toxicity, for example, it damages the hemotopoietic organs, alimentary tract and decreases immune function. The toxicity of other anticancer medicines, for example, Fluorouracil, Mustine and 6-Mercaptopurine are more heavy than Cyclophosphamide.

Some antibiotics are effective anticancer drugs, for example, Adriamycin is used for treatment of some cancers which include leukemias, gastric pancreatic and breast cancers. A prime limiting factor of the administration of adriamycin is cardiotoxicity. The most serious side effect of adriamycin administration is myocardial degeneration causing congestive heart failure.

This acute cardiomyopathy may manifest pericarditismyocarditis, acute left ventricular dysfunction, arrhythmia and myocardial infarction. Adriamycin induced cardiomyopathy was thought to be permanent and rapidly progressive. Late cardiomyopathy may develop in weeks, months or even years. Some patients were reported who developed progressive cardiomyopathy two and two-half years after receiving this drug.

Obviously, to overcome the toxicity of anticancer drugs is very important. Many agents have been suggested to reduce or prevent toxicity of anticancer medicines. For example, Vitamin E and N-acetyl-L-Cysteine have been reported to prevent adriamycin cardiotoxicity. Although more recent research appears to contradict these findings, both vitamin E and N-acetyl-L-Cysteine do not prevent adriamycin induced cardiotoxicity.

So far, no drugs have been succeeded to overcome the toxicity of anticancer drugs.

For the reasons given above, treatment of cancer is very difficult.

For the above reasons there is a need to provide a composition comprising several active ingredients which, in combination, are useful in treating and preventing toxicity of anticancer drugs in the human body and in increasing immune function. This invention provides such a pharmaceutical composition, referred to as PDI, as defined in claim 1. Further advantageous features are evident from the subclaims.

Specifically, this invention provides a new composition of four major active ingredients.

A. Ferulic acid extracted from Levisticum Officinale Koch or Angelica sinensis Diels.

B. Ginsenoside extracted from Panax quinquefolium L. or Panax ginseng C. A. Meyer.

C. Anethole extracted from Foeniculum Vulgare Mill.

D. Sodium cinnamate extracted from Cinnamomum Cassia Blume or Cinnamomum Cassia Presl.

The four herbs: Levisticum Officinale, Foeniculum Vulgare Mill, Penax quinquefoliumm L of Penax ginseng C. A. Myer and Cinnamomum Cassia Blume are regarded by the F.D.A. of U.S. as recognized as safe.

The following detailed description is to further describe the invention.

PDI comprises (A) Ferulic Acid, (B) Ginsenoside, (C) Anethole, (D) Sodium Cinnamate. The composition of PDI and the sources of its components are listed below:

TABLE 1

| Component | Source |
|---|---|
| (A) Ferulic Acid | Roots of Levisticum Officinale Koch or Angelica Sinensis Diels |
| (B) Ginsenoside | Roots of Penax quinquefolium L or Panax ginseng C. A. Meyer |
| (C) Anethole | Fruits of Foeniculum Vulgare Mill |
| (D) Sodium Cinnamate | Stem of Cinnamomum Cassia Blume or Cinnamomum Cassia Presl |

The process for producing PDI comprises extracting the ground form of the above natural materials with appropriate solvents such as alcohol or water, removing lipids by extraction with ether or petroleum ether; crystallisation or chromatographic fractionation are used in preparing PDI by mixing its components in the desired proportions.

In the following, the chemical structures of the components of PDI are shown

(I) Chemical Structure of Ferulic acid

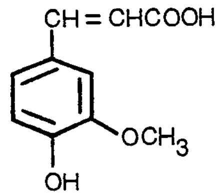

(II) Chemical structure of Ginsenoside

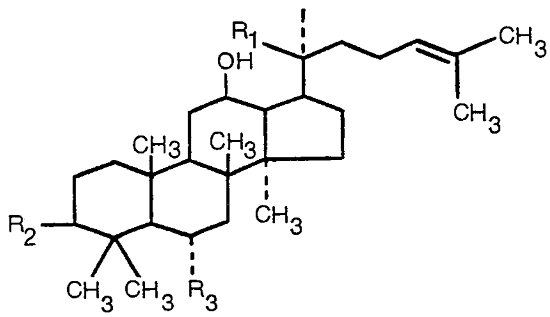

When

$R_1$ = Glucose 6 1 Glucose
$R_2$ = Glucose 2 1 Glucose
$R_3$ = H
The compound is Ginsenoside $b_1$.
Melting point, 198—202°C.

When

$R_1$ = O— Glucose
$R_2$ = OH
$R_3$ = —O— Glucose
The compound is Ginsenoside $g_1$.
Melting point, 192—194°C.

(III) Chemical structure of the glucose substituents of Ginsenoside

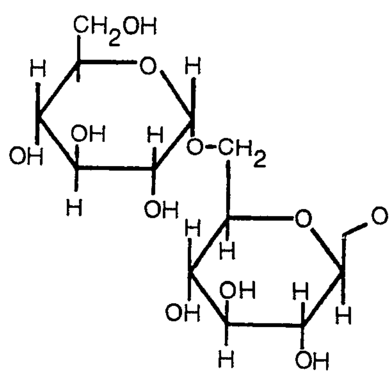

Glucose 6 1 Glucose

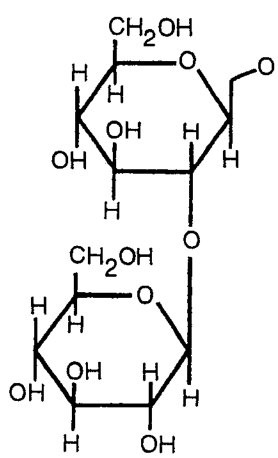

Glucose 2 1 Glucose

(IV) Chemical structure of Anethole

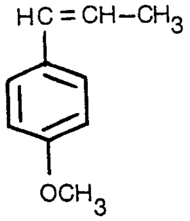

(V) Chemical structure of Sodium Cinnamate

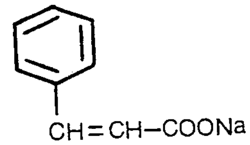

The invention is further illustrated by the following non-limiting examples and tests:

Example 1

Ferulic acid extracted from Levisticum Officinale Koch or Angelica sinensis Diels.

1 kg of dried powder of Levisticum Officinale Koch or Angelica sinensis Diels is extracted with 5000 ml of boiling water in 1 hour. Reapeat 3 times. Combine extracts. Distilled to 1000 ml by reduced pressure distillation at 22.65 mbar (17 mm Hg) is preferred. Add 2000 ml 95% ethanol to 1000 ml the residue with stirring and stand for 24 hours. Pour filter. Save the filtrate. Filtrate concentrated to syrup under reduced pressure distillation at 22.65 mbar (17 mm Hg) is preferred. Add 300 g silica gel to the syrup. Cooled at room temperature. Dried at 60°C. The dried powders are put into a percolator and washed with 95% ethanol until the ethanol becomes light colored. Ethanol is combined and distilled under reduced pressure 22.65 mbar, (17 mm Hg) is preferred. Ethanol is recovered and a still residue is obtained. This still residue is dissolved in 200 ml of distilled water. This water solution is mixed with polyamide. Set column. Wash with distilled water, then wash with 50% ethanol. 50% ethanol is combined and distilled at 22.65 mbar (17 mm Hg). The residue is dissolved in 100 ml of methanol. To this methanol is added 5 g silica gel. Dried. Set column (4 × 22 cm). Wash column with benzol — acetone (7:3). The benzol-acetone is combined and distilled at 22.65 mbar (17 mm Hg). Crystals are obtained and recrystallised in chloroform-methanol (1:1). White crystals are obtained.

Melting point is 169.5—171°C. Yield is about 0.1%.

Example 2

Extraction and Purification of Ginsenoside

1000 g of dried ginseng powder is extracted with 2000 ml of 95% ethanol at room temperature for 24 hours. The powder is recovered by filtration. Filtrate A is saved and the powder filtercake is refluxed with an additional 2000 ml of 95% ethanol on steam bath. The mixture is filtered again. Filtrate B is saved and the powder filtercake is refluxed twice more for 6 hours with additional 2000 ml batches of 95% ethanol and filtered, providing filtrates C and D. Filtrates A, B, C, and D are combined and distilled at reduced pressure (22.65 mbar (17 mm Hg) is preferred, whereby ethanol is recovered and a still residue is obtained.

This still residue is dissolved in 500 ml of distilled water. This water solution is extracted five times with 500 ml of lipophilic solvent, e.g. diethyl ether or petroleum ether, whereby lipids are removed from the solution.

To this aqueous raffinate is added 500 ml of watersaturated n-butanol and the mixture is distilled at reduced pressure 22.65 mbar (17 mm Hg) is preferred to dryness, whereby a powder residue is obtained. This powder is dissolved in 500 ml of anhydrous ethanol, and 2000 ml of acetone are added with agitation while a precipitate forms. The precipitate is recovered by filtration and washed twice with acetone and twice with diethyl ether and dried. About 60 g of a white to light yellow powder are recovered. This is Ginsenoside.

Example 3

Anethole extracted from Fennel (Foeniculum Vulgare Mill).

Take 1 kg Fennel oil. Fractonal-distillation by oil bath in distillation apparatus. Collect the distillate at 229° ~ 237°C. The resulting light-yellow liquor is anethole. $d_{25}^{25}$ 0.983—0.987. Refractive indec $n_D^{25}$ 1.588—1.561. Yield is 60%.

Example 4

Sodium Cinnamate

The Sodium Cinnamate is commercially available. The Sodium Cinnamate which is extracted from Cinnamomum Cassia Blume or Cinnamomum Cassia Presl is expensive.

Example 5

Preparation of PDI

On a dry basis, the composition of PDI may vary as follows:

TABLE 2

| | Weight percent | Preferred composition weight percent |
|---|---|---|
| Ferulic acid | 15—60 | 25 |
| Ginsenoside | 5—50 | 20 |
| Anethole | 20—65 | 30 |
| Sodium Cinnamate | 15—60 | 25 |

The dry ingredients of PDI, prepared in accordance with the present invention, may be incorporated in tablets, capsules and syrups by conventional methods.

Each oral dose for an adult is preferably 20 mg (20—200 mg) injected dose for an adult is preferably 10 mg (5—50 mg).

This invention will now be described with reference to its beneficial effects, as illustated by the following tests:

Example 6

The influence of PDI on the survival rate of myocardial cell

Materials and methods:

Hearts were removed from 11 days chicken embryos and were dissociated at 37°C for 45 minutes with 0.25% trypsin (sigma, type III), 0.025% collagenase (sigma, type I), and 0.005% pancreatin (NBCo) prepared in calcium and magnesium free saline G containing 4% chicken serum. Then the tissue was dispersed into a single cell suspension in culture medium containing 5 g/ml DNAse I(sigma). Viable cell counts were determined by hemocytometer counting. Cells were dispersed into 60 mm culture dishes (surface area 2000 $mm^2$) at densities of 200 cells/$mm^2$.

Culture were maintained in Ham's F—12K $cln^-$ modified by Clark [1981] and supplemented with 5% fetal bovine serum, gentamicin (5 mg/100 ml). Tissue culture plates were incubated under constant 5% $CO_2$ and 95% air at 37°C.

Cells counted — all cells were counted in 20 randomly selected fields across the entire dish. A Zeiss microscope 25 × objective having a field of view of 0.32 $mm^2$ was used for cell counting.

Immunostaining

I. Reagents

1. CMF solution: 8.0 g NaCl, 0.4 g KCl, 0.15 g $KH_2PO_4$, 0.29 $Na_2HPO_4 \cdot 7H_2O$, 1000 ml distilled water, solution is sterilized by passage through a 0.22 milliopore.
2. Phosphat-buffered saline (PBS): 10 ml 1M $PO_4$ buffer, 8 g NaCl, $H_2O$ 1 liter, pH to 7.6.
3. PBS/BSA: PBS plus 1% bovine serum albumin.
4. Biotinylated Antibody (BA): ABC kit, 1 drop BA in 20 ml PBS/BSA.
5. Avidin Biotin Complex, ABC stain: ABC kit, 2 drops; Avidin DH, 2 drops Biotin-peroxidase, 10 ml PBS/BSA.
6. Diaminobenzidine substrate, DAB: 5 mg diaminobenzidine tetrahydrochloride, 10 ml PBS, 0.004 ml 30% $H_2O_2$.
7. 0.3% $H_2O_2$ in methanol: 0.1 ml 30% $H_2O_2$ in 10 ml.
8. ABC kit purchased from Vector Laboratories, Burlingame, CA.
9. Adriamycin purchased from Adria Laboratories, Inc., Columbus, OH.

II. Immunostaining procedure

Monoclonal antibodies (diluted 1:1000 in PBS/BSA) were used Immunostaining procedure uses a modified method of the vectastain ABC immunoperoxidase staining procedure, briefly

1. To culture plates antibody of cardiac myosin were added before 3 hr for staining. Then the culture plates were continuously incubated for 3 hr at 37°C.
2. The cell culture plates were rinsed in CMF saline.
3. The sections were fixed for 5 min in 50% methanol and 50% acetone.
4. Rinsing in methanol was repeated 3 times.
5. The sections were incubated for 10 min in 0.3% $H_2O_2$ in methanol.
6. The sections were incubated for 30 min in BA antibody solution.

7. The sections were rinsed twice in PBS for 5 min each.
8. The sections were incubated in ABC stain for 1 hr.
9. The sections were rinsed three times in PBS for 5 min each.
10. Sections were incubated in DAB-peroxide substrate solution for 5 min.
11. The sections were rinsed for 10 min in water.
12. Counter-staining was performed for 30 sec in 1% aqueous fast green, then 3 times soaking in distilled water.
13. The cell cultures were dried at the air.

Cell were determined by recording the color and the number of microscopic fields (250 ×). Normal cells are light green and dead cells are brown or very dark green.

Results

The influence of PDI on adriamycin induced damage of chick myocardial cell:

After chick myocardial cell was put into the culture for 4 days, Adriamycin was added and remained in the culture for 24 hours. Then ABC immunoperoxidase was used to determine survival rate. Under the above condition it is clearly shown that PDI increase the survival rate of chick myocarcial cell.

TABLE 3

The influence of PDI on the survival rate of myocardial cell

Chick myocardial cells were put in culture for 4 days. Then Adriamycin was added. The concentration of each dish was 10 μg Adriamycin/ml medium. In the PDI group, PDI was added. In the Adriamycin group, an equal value of CMF solution was added. After 24 hours the survival rate was determined.

| | Survival rate (living/total × 100%) |
|---|---|
| Adriamycin (10 μ) | 36.3 ± 3.1 (*16) |
| Adriamycin (10 μ) + PDI | 61.0 ± 3.0 (*15) |
| | P 0.001 |

* indicate number of sampling
Concentration of PDI is 150 g.

Example 7

Effects of PDI of hemopoietic system

Effects of PDI on hemopoietic system were investigated. Results showed that PDI (ip) could markedly improve the recovery rate of hemopoieses in mice treated with cyclophosphamide.

With an increased number of nucleated cells in bone-marrow (BMC), endogenous colonies in spleen and higher $^3$H-TdR uptake in marrow and spleen can be served. The level of serum colony stimulating factor (CSF) increased after injection. It is found that PDI protect the stem cells of bone marrow in mice from the killing effect of cyclophosphamide.

Method of animal model is regular which is not part of this invention. Pharmacological effects are as illustrated by the following table: by means of the spleen colony assay technique the action of PDI on bone marrow stem cells (CFU—S) is shown.

TABLE 4

| Group | Number of sample | Means CFU—S ± SD | P |
|---|---|---|---|
| Control | 10 | 10.0 ± 0.81 | — |
| Cyclophosphamide | 10 | 4.0 ± 0.71 | < 0.001 |
| PDI + Cyclophosphamide | 10 | 7.7 ± 0.80 | > 0.05 |

Experimental procedure:

Male mice weighing 18—20 g were used in the experiments and were divided into treated (PDI) and control groups. The dosage of PDI was 5.5 mg/kg injected intraperitoneally. The control mice were injected with the same volume of normal saline. These injections were repeated daily for 3 days. On the last day,

both treated and control group cyclophosphamide were injectected intaperitoneally. The dosage of cyclophosphamide was 4.5 mg/kg.

The experimental procedure of other examples with mice are similar to the above procedure.

Example 8

The effect of PDI on phagocytosis of peritoneal macrophage of mice.

Method

Add 0.02 ml of 5% washed chick red blood cells suspension to 0.5 ml of the peritoneal exudate, shake gently to mix and incubate at 37°C for 5 minutes. Dip two coverslips, close to each other, in the above mixture and incubate for 30 minutes for the migration of the macrophages along the cover slips, fix and stain with Sharma stain. Examine microscopically for:

Phagocytic rate — number of macrophages with phagocytized chick red blood cells per 100 macrophages counted.

Method of animal model is regular.

Results

Pharmacological effects are as illustrated by the following table.

TABLE 5

| Group | Number of sample | Phagocytic rate ± SD(%) | P |
|---|---|---|---|
| Control | 10 | 35.10 ± 2.01 | — |
| Cyclophosphamide | 10 | 8.00 ± 0.36 | < 0.001 |
| Cyclophosphamide + PDI | 10 | 33.00 ± 1.14 | > 0.05 |

* concentration of PDI and cyclophosphamide is the same as in example 7.

Example 9

The effect of PDI on white blood cells in rats treated by cyclophosphamide

Action of PDI and cyclophosphamide on white blood cells was investigated by means of white blood cells assay. It was revealed that PDI protect white blood cells in rats from the killing effect of cyclophosphamide. Method of animal is regular. the dosage of PDI and cyclophosphamide is likey in example 7. Time of treatment is 10 days. The results are listed in the below table:

TABLE 6

| Group | White blood cells $\times 10^3/cm^3 \pm SD$ | Number of Sample | P |
|---|---|---|---|
| Normal | 14.6 ± 2.1 | 10 | — |
| Cyclophosphamide | 5.3 ± 1.0 | 10 | < 0.001 |
| Cyclophosphamide + PDI | 12.0 ± 1.8 | 10 | > 0.05 |

Example 10

The effect of PDI on lymphoblastoid transformation:

By means of the $^3H$—TdR liquid scintillation assay technique, the action of PDI on lymphoblastoid transformation was investigated by the following method:

(1) Experimental procedure of animal is like example 7.

(2) Lymphoblastoid transformation test:

I. Reagents and conditions for cells culture

a. Culture media — RPMI 1640, medium 199 minimal essential medium (Eagle).

b. Buffer — Hepes buffer, the final concentration at 37°C was 25 mM, to maintain the pH of the medium at 7.31.

c. Serum — generally 15—20% fetal bovine serum was incorporated, or lymphocytes from mice, 5% was used.

d. Gaseous phase — 5% $CO_2$ in air.
e. Cell concentration — generally 1—2 × $10^6$/ml
f. Stimulants — 20 µl/ml for phytohemagglutinin containing polysaccharide (PHA—M) or 10 µl/ml for polysaccharide-free purified phytohemagglutinin (PHA—P).

II. Measurement by liquid scintillation

a. The conditions of the cell culture are the same as above. 3H—TdR was added after 48 hours of incubation at a final concentration of 1 µCi/ml. The incubation were continued for 24 hours.

b. Wash the cells twice with cold normal saline and the erythrocytes were lysed by addition of distilled-water and an equal volume of 3.6% NaCl was then added. The intact lymphocytes were again washed once with cold saline. The lymphocytes were spinned down 2 ml of 10% trichloroacetic acid were added to precipitate the protein and washed twice with normal saline. 2 ml of ethanol:ether (1:1) were added to wash one. 0.2 ml of formic acid was then added for digestion till the precipitate was dissolved.

c. 4 ml of scintillation fluid were added to 0.1 ml of the final sample and were counted in a liquid scintillation counter.

Results are listed in the table below: (concentration of PDI an cyclophosphamide is the same as in example 7.)

TABLE 7

| Group | Number of sample | CPM ± SD | P |
|---|---|---|---|
| Normal | 10 | 1340 ± 51 | — |
| Cyclophosphamide | 10 | 697 ± 38 | < 0.001 |
| Cyclophosphamide + PDI | 10 | 1353 ± 50 | > 0.05 |

Example 11
Toxicity of PDI

1. L.D.$_{50}$: 1321 mg/kg when injected in to the abdominal cavity of mice.

2. Each dose for an adult is 20 mg. Using 50 kg as the average weight of an adult the dosage is 0.4 mg/kg, therefore it is very safe.

The results and experimental procedure of PDI to overcome toxicity of radiotherapy is like for the above described chemotherapy.

The embodiment of the invention described here can be modified within the spirit and scope of the present invention. Numerous modifications and variations of the present invention are possible in the light of the above teachings.

**Claims**

1. A pharmaceutical composition for decreasing side effects of anticancer chemotherapy and for increasing the immune function comprising, in weight percent: Ferulic acid 15—60%; Ginsenoside 5—50%; Anethole 20—65% and Sodium Cinnamate 15—60%.

2. The pharmaceutical composition according to claim 1, in which the Ferulic acid is prepared by:

(a) extracting the powder of Levisticum Officinale Koch or Angelica Sinensis Diels with water and adding ethanol to the aqueous extract;

(b) adding silica gel to the water/ethanol extract, drying the whole mixture to a powder, extracting the powder with ethanol and dissolving the extracted material in distilled water;

(c) adding polyamide to the aqueous solution and carrying out a chromatographic fractionation of the same;

(d) crystallizing Ferulic acid in benzol-acetone and recrystallizing the crystals in chloroform-methanol.

3. The pharmaceutical composition according to claim 1 or 2, in which the Anethole is prepared by separating Anethole from Fennel oil by fractional distillation.

**Patentansprüche**

1. Pharmazeutische Zusammensetzung zur Verminderung von Nebenwirkungen der Antikrebschemotherapie und zur Erhöhung der Immunfunktion mit, in Gew.-%, 15—60% Ferulasäure, 5—50% Ginsenosid, 20—65% Anethol und 15—60% Natriumcinnamat.

2. Pharmazeutische Zusammensetzung nach Anspruch 1, in der die Ferulasäure durch

a) Extrahieren des Pulvers von Levisticum officinale Koch oder Angelica sinensis Diels mit Wasser und Zugeben von Ethanol zu dem wässrigen Extrakt,

b) Zugeben von Kieselgel zu dem Wasser/Ethanol-Extrakt, trocknen der gesamten Mischung zu einem Pulver, Extrahieren des Pulvers mit Ethanol und Lösen des extrahierten Materials in destilliertem Wasser,

c) Zugeben von Polyamid zu der wässrigen Lösung und Durchführen einer chromatographischen Fraktionierung derselben,

d) Kristallisieren der Ferulasäure in Benzol-Aceton und Umkristallisieren der Kristalle in Chloroform-Methanol hergestellt ist.

3. Pharmazeutische Zusammensetzung nach Anspruch 1 oder 2, in der das Anethol durch Abtrennen des Anethols aus Fenchelöl mittels fraktionierter Destillation hergestellt ist.

**Revendications**

1. Composition pharmaceutique destinée à réduire les effets secondaires de la chimiothérapie anticancéreuse et à accroître la fonction immune, comprenant, en pourcentage en poids: 15 à 60%, d'acide férulique, 5 à 50% de ginsénoside, 20 à 65% d'anéthol et 15 à 60% de cinnamate de sodium.

2. Composition pharmaceutique suivant la revendication 1, dans laquelle l'acide férulique est préparé:

(a) en soumettant à une extraction la poudre de Levisticum officinale Koch ou Angelica sinensis Diels avec de l'eau et en ajoutant de l'éthanol à l'extrait aqueux;

(b) en ajoutant du gel de silice à l'extrait aqueux/éthanolique, en déshydratant le mélange total pour former une poudre, en soumettant la poudre à une extraction avec de l'ethanol et en dissolvant la matière extraite dans de l'eau distillée;

(c) en ajoutant un polyamide à la solution aqueuse et en effectuant un fractionnement chromatographique de cette solution;

(d) en provoquant la cristallisation de l'acide férulique dans un mélange benzol-acétone et une recristallisation des cristaux dans un mélange chloroforme-méthanol.

3. Composition pharmaceutique suivant la revendication 1 ou 2, dans laquelle l'anéthol est préparé en séparant l'anéthole de l'essence de fenouil par distillation fractionnée.